# EUROPEAN PATENT SPECIFICATION

(11) **EP 3 229 882 B1**
(45) Date of publication and mention of the grant of the patent: **07.08.2019**
(21) Application number: 15825881.4
(22) Date of filing: 08.12.2015
(51) Int. Cl.: A61M 16/06

(54) **A HYBRID POSITIVE AIRWAY INTERFACE SYSTEM FOR USE WITH VENTILATION AND POSITIVE AIR PRESSURE SYSTEMS**
HYBRIDES POSITIVES ATEMWEGSSCHNITTSTELLENSYSTEM ZUR VERWENDUNG MIT BEATMUNGS- UND POSITIVLUFTDRUCKSYSTEMEN
SYSTÈME D'INTERFACE POSITIVE ET HYBRIDE DES VOIES RESPIRATOIRES À UTILISER AVEC DES SYSTÈMES DE VENTILATION ET DES SYSTÈMES À PRESSION D'AIR POSITIVE

(30) Priority: 08.12.2014 US 201462088825 P
(43) Date of publication of application: 18.10.2017
(73) Proprietor: Human Design Medical, LLC, Allston, MA 02134 (US)
(72) Inventor: HARRISON, Donald, Charlestown, Massachusetts 02139 (US)
(74) Representative: Appleyard Lees IP LLP
(86) International application number: PCT/US2015/064536
(87) International publication number: WO 2016/094429

(56) References cited:
- EP-A2- 2 327 442
- WO-A1-2008/040050
- WO-A1-2013/142909
- WO-A1-2014/183167
- WO-A2-2014/155329
- US-A1- 2006 174 887
- US-A1- 2007 125 385
- US-A1- 2014 283 842

## Description

### BACKGROUND OF THE INVENTION

### 1. Field of the Invention

The present invention relates to medical devices, and, more particularly to masks and headgear portions of air delivery devices that assist with the delivery of gas to the breathing airways of users. These mask and headgear systems and devices can be used with positive airway pressure [PAP] such as continuous positive airway pressure devices [CPAP], automatic positive airway pressure devices [APAP], variable positive airway pressure devices [VPAP], and bi-level positive airway pressure devices [BPAP].

### 2. Description of the Prior Art

Previous masks have been bulky, heavy, and/or difficult to secure over a user's breathing airways. Additionally, the pressure on and around the face required to maintain a bulky nasal mask system in place can be uncomfortable. Additionally, numerous prior art systems seek to cover only the mouth or only the nose which can often be problematic for users who regularly switch between mouth and nasal breathing during sleep. Previous systems which cover both nose and mouth, often being overly bulky or otherwise cumbersome to wear.

US 2014/283842 A1 is background art and discloses an interface comprising a nasal sealing portion. US 2007/125385 A1 is background art and discloses a full face respiratory mask with integrated nasal interface.

WO 2013/142909 A1 is background art and discloses a patient interface for treatment of a user having a respiratory disorder. EP 2 327 442 A2 is background art and discloses a sealing nasal cannula. WO 2014/183167 A1 is background art and discloses an oro-nasal patient interface to provide breathable gas to a patient.

The present invention seeks to address these concerns by providing a lighter weight mask system that is secured around the breathing airways of the user using a lower retention force while maintaining a good seal for use with PAP devices. The mask system can include a mask assembly system that has interchangeable components, is light-weight, and provides a uniform sealing around an area surrounding a user's nares and mouth that is enabled by a unique internal support structure and nasal pillow extension assembly.

### SUMMARY OF THE INVENTION

Provided is a positive airway interface system as set out in the accompanying claim 1.

In yet additional embodiments the sealing membrane can be affixed to the support structure of the mouth interface by interferingly engaging a channel provided about a circumference of a facial aperture of the mouth interface.

In some embodiments a removable heat moisture exchange can be provided and disposed within the positive air pressure adapter.

In yet additional embodiments the system can further include one or more CO2 washout vents being provided through the positive air pressure adapter, wherein the CO2 washout vent(s) are provided in a removable insert which covers a front aperture of the positive air pressure adapter.

In yet additional alternative embodiments the positive airway interface system can further include an anti-asphyxia valve provided through the positive air pressure adapter.

In yet additional embodiments the positive airway interface system can further include a plurality of mouth interface attachment mechanisms mechanically coupled to the support structure of the mouth interface. These attachment mechanisms can be formed of a plurality of circular grommets being configured to affix to corresponding loops of a headgear assembly.

In yet additional embodiments the positive airway interface system can further include a first extension disposed on a first end of the nasal interface; and a second extension disposed on a second end of the nasal interface as well as a plurality of strap assemblies, including a first strap assembly configured to couple to the mouth attachment mechanisms and a second strap assembly configured to couple to the first and second nasal attachment mechanisms, the plurality of strap assemblies configured to provide a multidimensional sealing force between the positive airway interface system and the user's face. In such an embodiment the one or more strap assemblies can be configured to couple to the mouth attachment mechanisms and the first and second nasal attachment mechanisms, wherein the one or more strap assemblies are configured to position the positive airway interface system about the user's face.

In yet additional embodiments the positive airway interface system can further include a first malleable sealing membrane which extends inwardly into an internal cavity of the facial mask from an external edge of the internal support structure, as well as a second malleable sealing membrane extending inwardly into the internal cavity of the facial mask from an internal edge of the internal support structure.

In yet additional embodiments the positive airway interface system can further include a support membrane extending from a first point of an outer circumference of the facial mask to a second point of the outer circumference of the facial mask, the support membrane configured to rest on a maxilla portion of the user's face between the user's nose and mouth.

These and other embodiments are described in more detail herein.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 illustrates a front view of a user wearing a hybrid positive airway interface system in accordance with various aspects of the present invention
FIG. 2 illustrates a perspective view of the hybrid positive airway interface system of FIG. 1;
Fig. 3 illustrates a partial perspective exploded view of the hybrid positive airway interface system perspective view of FIG. 1;
FIGs. 4A-C illustrate front and rear perspective views as well as a side cross sectional view of a mouth interface for use with the hybrid positive airway interface system of FIG. 1;
FIGs. 5A-B illustrate front and rear perspective views of a rigid adapter for use with the hybrid positive airway interface system of FIG. 1;
FIGs. 6A-B illustrate front and rear perspective views of an input adapter for use with the hybrid positive airway interface system of FIG. 1;
FIG. 7 illustrates a side perspective view of an exemplary heat moisture exchange (HME) for use with the hybrid positive airway interface system of FIG. 1;

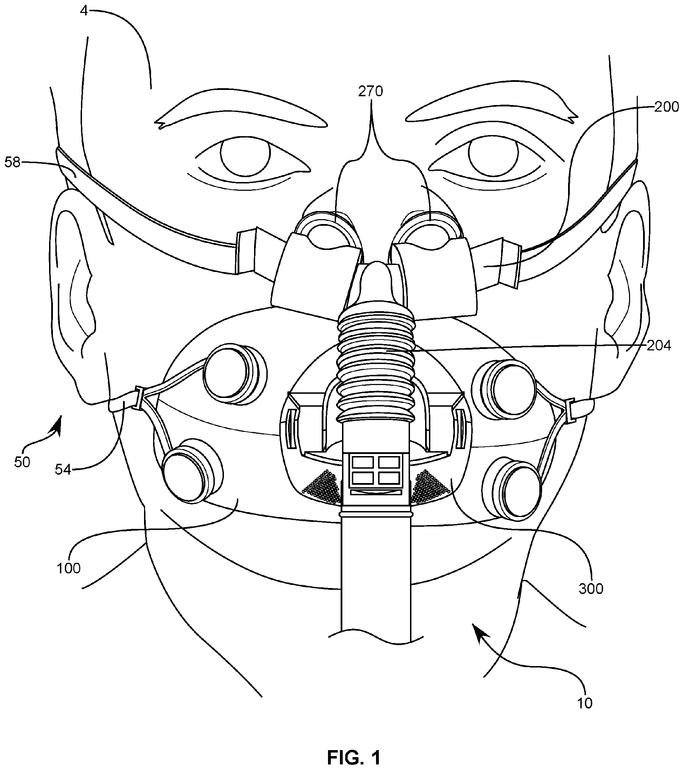

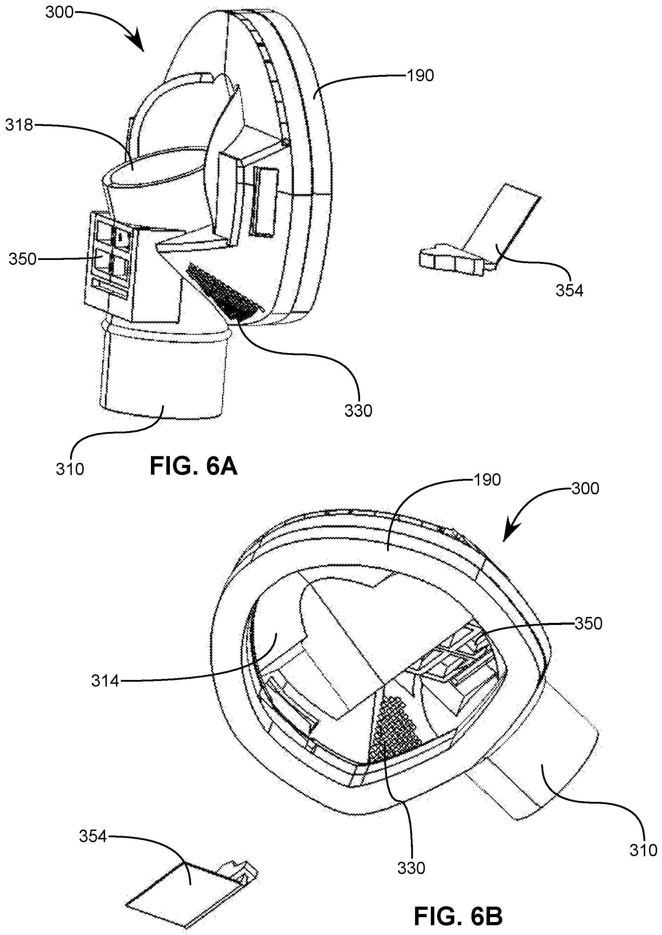

FIGs. 6A-B illustrate rear internal views of a mouth interface for use in the embodiment of FIGs. 1A-B;

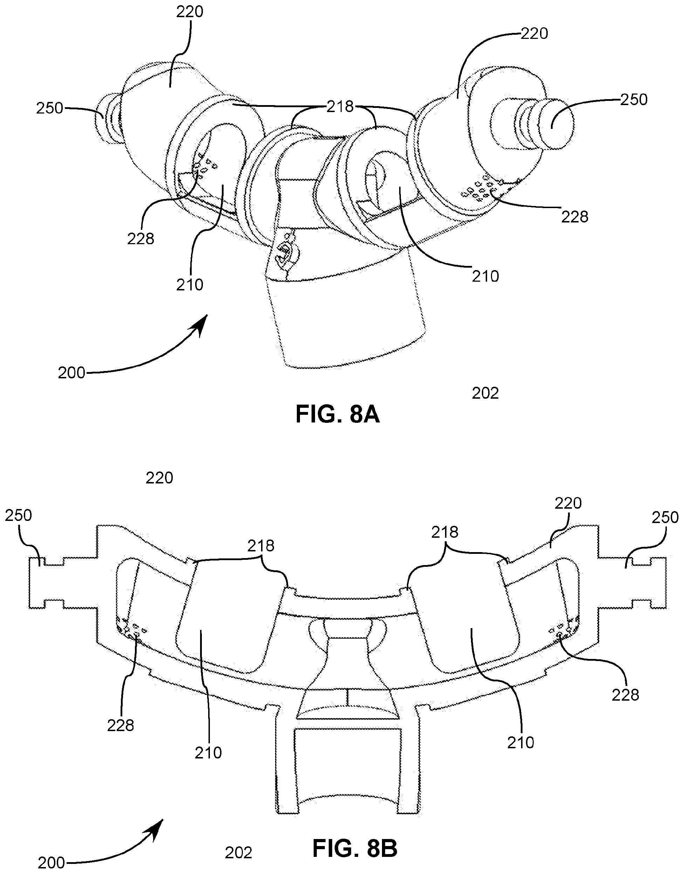

FIGs. 8A-B illustrate front perspective and front cross-sectional views of a nasal interface for use with the hybrid positive airway interface system of FIG. 1;

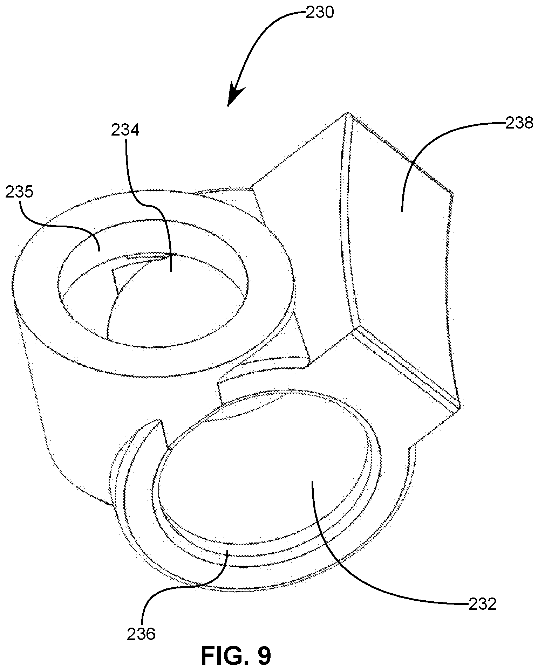

FIG. 9 illustrates a side perspective view of an exemplary nasal pillow interface for use with the nasal interface system of FIGs. 8A-B; and

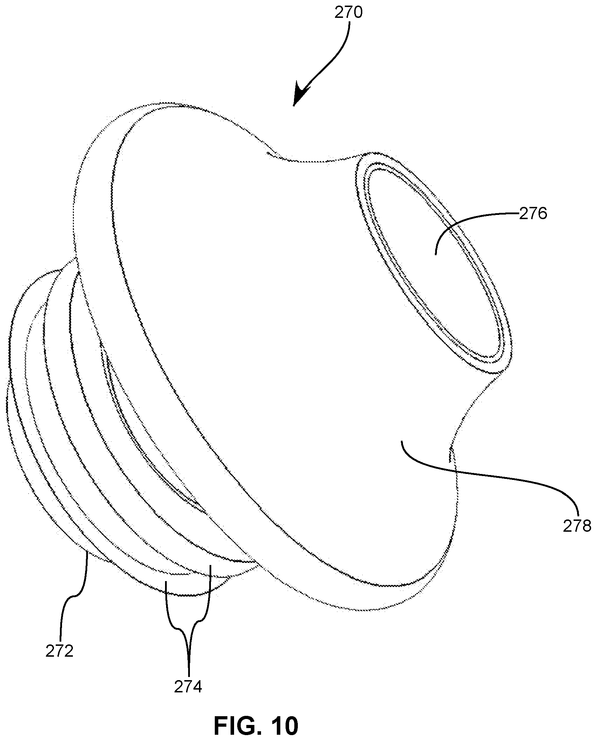

FIG. 10 illustrates a side perspective view of an exemplary nasal pillow for use with the nasal interface system of FIGs. 8A-B.

### DETAILED DESCRIPTION OF THE PREFERRED EMBODIMENT

To provide an overall understanding of the systems, devices, and methods described herein, certain illustrative embodiments will be described. Although the embodiments and features described herein are frequently described for use in connection with CPAP apparatuses, systems, and methods, it will be understood that all the components, mechanisms, systems, methods, and other features outlined below can be combined with one another in any suitable manner and can be adapted and applied to other PAP apparatuses, systems, and methods, including, but not limited to, APAP, VPAP, and BPAP apparatuses, systems, and methods.

The present application seeks to provide a solution to the aforementioned problems by creating an adjustable, comfortable, mask assembly system that has interchangeable components, is light-weight, and provides a uniform sealing around an area surrounding a user's nasal passages and mouth which is enabled by a unique internal support structure and nasal extensions.

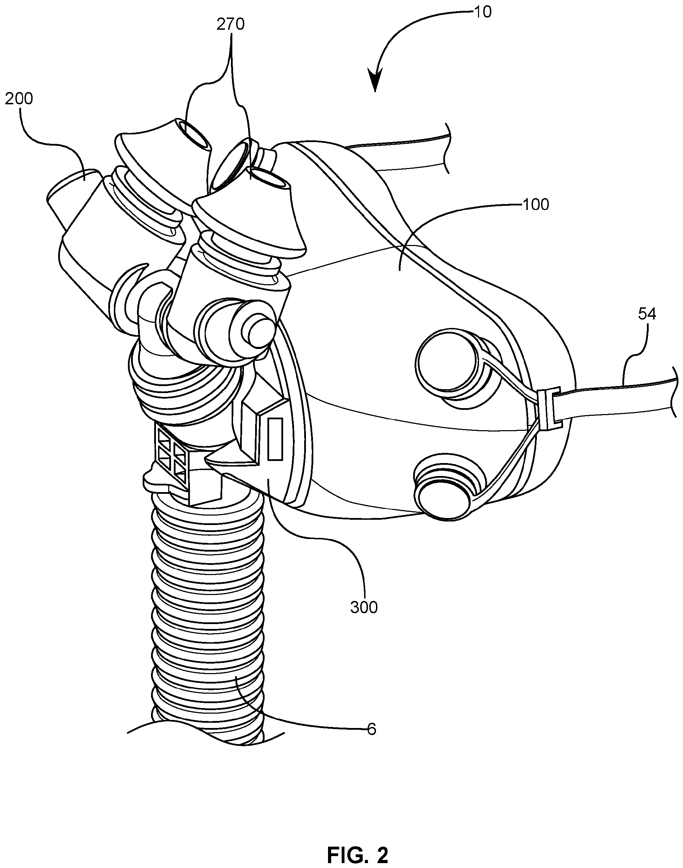

Figs. 1-2 illustrate a hybrid mask assembly 10, the hybrid mask assembly 10 having a mouth interface 100 and a nasal interface 200 fluidly connected to a positive pressure air supply source (not shown). The positive pressure air supply can be provided via various methods including a CPAP pump, blower, etc. (not shown), the method depicted here includes a supply hose 6 and an associated adapter 300, which can be fluidly connected to the positive pressure air supply. In this manner positive air pressure can be supplied into the hybrid mask assembly 10 through the adapter 300, and diverted into the mouth interface 100, as well as into the nasal interface 200 through a nasal adapter tube 204 and thus provide positive air pressure/flow into the airways of the user 4.

As shown, the mouth interface 100 can be configured to extend around the user's mouth and the nasal interface 200 can include a pair of nasal pillows 270 which interface with each of the user's nares/nostrils. In this manner the adapter 300 can be configured to provide positive air pressure from the positive air pressure source into both the mouth interface 100 and the nasal interface 200 simultaneously.

The hybrid mask assembly can be positioned about the user's face and the placement can be maintained using a strap assembly 50, wherein the strap assembly 50 can include a lower mouth strap 54 and an upper nasal strap 58. The mouth strap 54 and the nasal strap 58 can be adjusted independently so as to provide sufficient placement retention and sealing forces between the mouth and nasal interfaces and the face of the user.

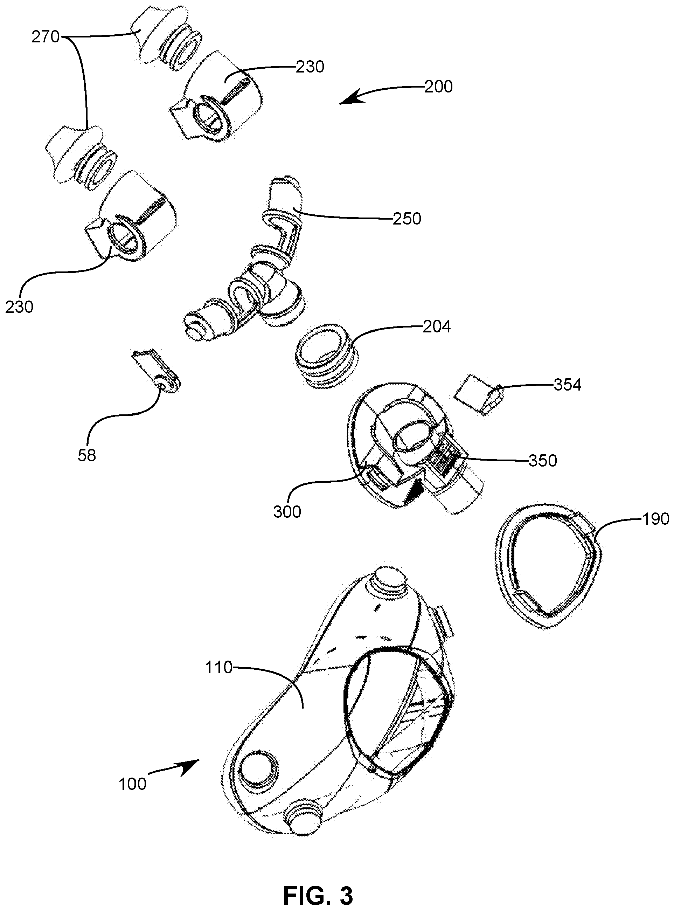

Fig. 3 illustrates an exploded view of the hybrid mask assembly 10 so as to better illustrate the various components and how they relate with one another. As discussed briefly above the adapter 300 can have an inlet which receives a pressurized supply of air. The adapter 300 can attach to the mouth interface 100 wherein the mouth interface includes a rigid coupler 190 which facilitates attachment of the adapter 300 to a semi-flexible mask portion 110. The adapter 300 can also couple to nasal interface 200 by means of a flexible adapter hose 204 which attaches to an upper portion of the adapter 300 and thus provides fluid communication to a nasal extension portion 250. The nasal extension portion 250 can include a pair of apertures over which can be provided a pair of corresponding nasal pillow interfaces 230 which can then interface with corresponding nasal pillows 270 so as to provide a positive air pressure supply to the user's nostrils.

It will be further appreciated that because the hybrid mask assembly interfaces over both of the user's breathing passageways, i.e. the nose and the mouth, wherein a fear of asphyxiation is presented should the positive air pressure source quit working. In order to counteract this potential risk the adapter 300 can include an aperture 350 and closure flap 354 which operate together to form an anti-asphyxia valve, wherein the anti-asphyxia valve is responsive to air flow, and can open in response to a zero pressurized air flow condition and then allow the user to breath air directly from the environment through the then opened anti-asphyxia valve. The specific functionality of this valve will be discussed in more detail below.

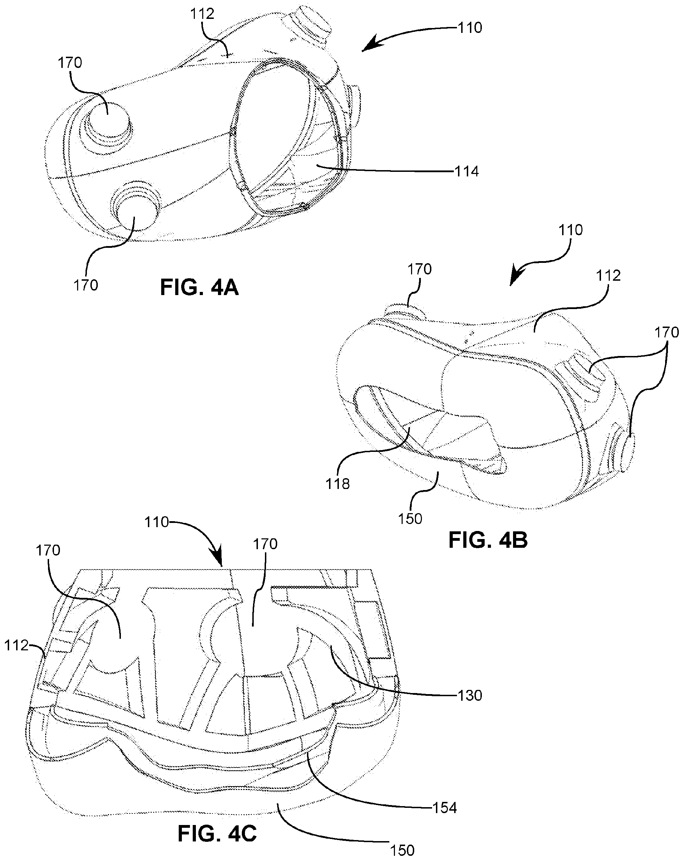

As discussed above, the mouth interface 100 can include a semi-flexible mask portion 110 which is configured to provide a seal around the user's mouth by transmitting a tensile sealing force applied the strap assembly 54, as shown in FIG. 1. As shown in FIGs. 4A-C the semi flexible mask portion 110 can include a plurality of attachment mechanisms 170 which extend through an outer shell and connect to an internal support structure 130. In this manner the tensile sealing force can be applied through the semi-flexible mask portion 110 and ultimately onto a sealing membrane 150. The sealing membrane 150 can extend from the outer shell 112 or the internal support structure 130 from an outer perimeter of the user interface aperture 118 toward a central portion of the user interface aperture 118. The sealing membrane can be formed of a thin and malleable substance so as to conform to the contours of the user's face. It will be appreciated that the semi-flexible mask portion 110 can be formed such that the mask is semi flexible, so as to provide a comfortable fit about the user's face, wherein the internal support structure 130 provides sufficient rigidity so as to not collapse under the sealing force applied by the strap assembly, but is not so rigid so as to cause uncomfortable pressure points about the user's face.

The internal support structure 130 operates to suspend and provide support to the relatively flexible and thin outer shell 112 which would otherwise have insufficient structural support so as to not collapse under the sealing force. This combination allows for a thinner outer shell 112 to be suspended between the interconnected truss or web structure of the internal support 130 and thus allows for a thinner exterior wall and ultimately a lighter and thus more comfortable mask.

In some embodiments the attachment mechanisms can be formed as a plurality of circular grommets 170 being configured to affix to corresponding loops of a mouth strap assembly as shown in FIG. 1. In this manner the connection between the circular grommets and the corresponding loops can have a rotational degree of freedom so as to increase the potential adjustability of the fit.

In yet additional embodiments the mask 110 can be provided with a plurality of malleable sealing membranes 150 and 154 being disposed about the user interface aperture of the nasal mask. The first malleable sealing membrane 150 can be provided so as to extend inwardly into an internal cavity of the mask from an external edge of the internal support structure 130 and the second malleable sealing membrane 154 can be provided so as to extend inwardly into the internal cavity of the mask from an internal edge of the internal support structure 130.

It will then be further appreciated that because the semi-flexible mask portion 110 is deformable to a certain extent it can thus be difficult to securely couple to, or in other words it will then be easy to pull away from, the otherwise relatively rigid input adapter 300. For this reason, and as will be discussed in more detail below, a rigid coupler 190 can be provided between the input adapter 300 and the semi-flexible mask portion 110 so as to facilitate a more secure connection between the two.

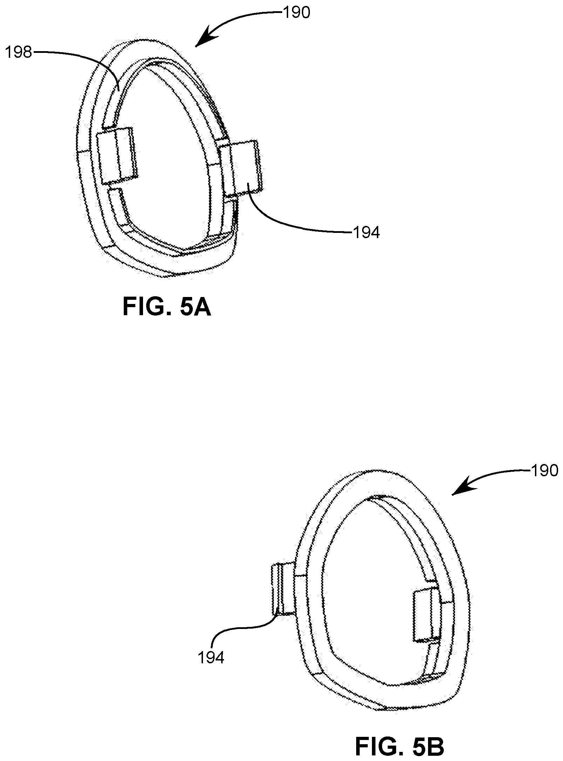

FIGs. 5A-B illustrate a rigid coupler 190 which provides a more secure coupling to the semi-flexible mask portion as shown in FIG. 3.

FIGs. 6A-B illustrate how the rigid coupler 190 provides a secure interface with the rigid input adapter 300. The rigid coupler 190 is intended to serve as a more secure attachment means between the inlet adapter 300 and the semi-flexible mask 110. As such the rigid coupler 190 can be permanently attached to, i.e. bonded to or unitarily formed with, the semi-flexible mask 110, or alternatively the rigid coupler 190 can be removably provided to the semi-flexible mask 110 and a reliable seal provided therebetween. In some embodiments the rigid coupler 190 can be provided with a raised protrusion 198 which can interface with a corresponding channel on either the mask or the input adapter so as to provide an air tight seal therewith.

The rigid coupler 190 can further be provided with clip 194 for interfacing with corresponding clip receivers of the inlet adapter 300, or alternatively the rigid coupler can be provided with clip slots (not shown) which can merely be pass through slots allowing the clip protrusions of the inlet adapter to interface directly with the corresponding clip interfaces of the mask itself.

Additionally, the mask 110 can be provided with a flat or otherwise consistent bonding surface for bonding to the rigid coupler 190 using adhesives or other suitable bonding agents. Alternatively, the mask 110 can be overmolded over the rigid coupler 190 so as to integrate the coupler 190 into the inlet aperture 114. If the rigid coupler 190 is formed unitarily or overmolded by the mask 110 it will then be unnecessary for both the mask and the rigid coupler to have corresponding clip interfaces.

FIGs. 6A-B illustrate a rigid input adapter 300 for use in the hybrid mask assembly discussed above. The rigid input adapter 300 can include an input 310 configured to receive a supply of pressurized air from a pressurized source. The flow of pressurized air enters an internal cavity and then exits the input adapter 300 from various outlets 314 and 318. The outlet 314 being configured to couple to the mouth interface (not shown) and the outlet 318 configured to couple to the nasal interface (not shown).

Additionally, the input adapter 300 can include a plurality of micro apertures 330 being covered by or provided as a carbon dioxide permeable membrane, such as a silicon knife coating, which allows carbon dioxide to pass therethrough, such that carbon dioxide can escape as from the mouth interface into the surrounding environment as the user exhales.

The views depicted in FIGs. 6A-B also illustrate how the flap 354 and aperture 350 operate as an anti-asphyxia valve to prevent asphyxiation of the user if the blower stops operating. The inlet adapter 300 can be provided with an aperture 350 having a flat interior surface corresponding in shape to the flap 354. The valve flap 350 can be attached to the input adapter 300 and be configured to selectively close the aperture 350 in response to air flow through the inlet adapter 300. For example, flowing air coming from the tube adapter will push the flap upwards, thus pressing the flap against the flat interior surface and thereby closing the aperture 350, however, when air is not flowing, the flap 254 can fall downward so as to open the aperture 350 and thus allow the user to breathe fresh non-pressurized air from outside the mask through the aperture.

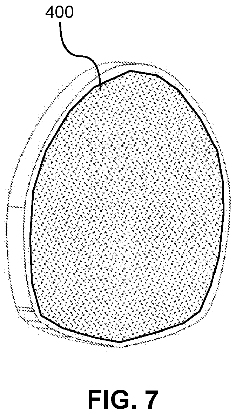

In some embodiments, and as shown in FIG. 7, a heat moisture exchange (HME) can be provided in the form of a porous member or membrane 400. The HME 400 can be formed of varying materials, porous, wicking, or otherwise so as to retain exhaled moisture within the mask 110 or within the input adapter 300. This HME 4000 can be shaped so as to correspond in shape to the interior of the mask or input adapter and cover or force the air supply to pass therethrough such that the trapped moisture can then be partially evaporated or entrained into the inlet air supply and thus increase the moisture content of the air supply and reduce the harshness of the supplied air. This HME 400 can be formed in varying shapes and thicknesses so as to be adaptable for use in the input adapter disclosed herein, or alternatively, within the nasal interface.

FIGs. 8A-B illustrate isometric and cross-sectional front views of the nasal interface 200. The nasal interface 200 can be configured to receive positive air pressure directly from the positive air pressure adapter 300, and can be provided with trunk having an input 202 configured to connect to the flexible extension 20, as shown in FIG. 1 , so as to allow for a flexible interface between the positive air pressure adapter (not shown) and the nasal interface 200.

It will be appreciated that the flexible connector 204 allows for easy and independent adjustment of the nasal interface 200 with respect to the varying positioning or differences in relative placement possible between a user's nares and respective mouths which can vary greatly between users. It will be appreciated that various users will have different shaped noses with varying widths and lengths, and varying distances with respect to each individual's mouth. Having a flexible interface 204 which can be provided at varying lengths allows for a consistently comfortable fit between the nasal interface 200 and the mouth interface 100.

The nasal interface 200 can also be provided with a pair of extensions 220 that extend outwardly from a central trunk 202. The extensions 220 can be provided with a pair of apertures 210 over which respective right and left nasal pillow interfaces (not shown) can be provided which allow for the attachment of a pair of custom nasal pillows (not shown). The apertures can thus provide fluid communication from the flexible connector 204 and the input adapter (not shown) so as to provide positive air pressure through the apertures, to the nasal pillows, and into the nares of the user.

It will be appreciated that the extensions 220 can also be provided with CO2 vent holes or micro apertures 228 or other exhaust ports or holes as deemed necessary. These micro apertures can also be configured to allow carbon dioxide to pass therethrough, such that carbon dioxide can escape as from the nasal interface into the surrounding environment as the user exhales.

The extensions can further be provided with a strap interface about distal ends of each extension configured to attach to the nasal strap assembly. The strap interface can be provided as tabs, as shown which can allow for a radial adjustment between the strap and the respective distal end of each extension so as to facilitate greater adjustability and therefore comfort.

The apertures 210 can be provided with a pair of raised flanges 218 on either side about the distal edges of the apertures 210. The raised flanges 218 interface with corresponding internal flanges of the nasal pillow interface (not shown) which fits over the apertures 210 and provides a seal and thus transmits the pressurized air from the apertures to the nasal pillows.

FIG. 9 illustrates an exemplary nasal pillow interface 230, which fits over the apertures as shown in FIGs. 8A-B. The nasal pillow interface 230 includes an inlet aperture 232 which fits over and around the extension arms 220 of FIGs. 8A-B having an interior flange 236 which interfaces against and seals with the flange 218 as shown in FIGs. 8 A-B. This interface allows the pillow interface 230 to rotate axially about its respective extension arm so as to increase adjustability while maintaining an air-tight seal therebetween. The nasal pillow interface 230 also includes an outlet aperture 234 with an interior flange 235 configured to interface with corresponding flanges on the nasal pillow (not shown here). The nasal pillow interface 230 can further include a rest 238 which can be provided with a pad or other soft material wherein the rest 238 can be configured to rest on the user's face between the nose and the upper lip of the user.

FIG. 10 illustrates an exemplary nasal pillow 270 which can be formed having a round base portion, the base portion being provided with a plurality of flanges 274 about an inlet end 272. The nasal pillow 270 can be formed having an elliptical nasal cone 278 with an outlet aperture 276 which delivers the pressurized air flow to the interior of the user's nares/nostrils. The round base and elliptical cone allows the nasal pillow to be rotatable with respect to the nasal pillow interface 230 because the flanges 274 can interface with the interior flanges of the nasal interface outlet (not here shown) in a coaxially free manner. In this manner the user can rotate the ellipse to a comfortable position without breaking the seal. Additionally, a plurality of such flanges 274 can be provided along their axial length so as to allow for incremental height adjustment of the individual nasal pillows with respect to their corresponding nasal pillow interfaces.

It will be appreciated that individual users can have different sized noses and faces; thus, a need often exists to have various mask sizes. It will be appreciated that various sizes and shaped mask assemblies can be provided so as to better fit the user and customize the mask assembly 10 based on the user's individual preferences.

In some embodiments the sealing membranes 150 and 154 can be formed separately from the mask support structure 130 allowing for different membrane stiffness and size for between various membranes. It will be further appreciated that while the present embodiments show only a sealing membrane having a plurality of interior flaps 150 and 154 respectively, however, the hybrid mask assembly 10 can be provided with numerous flaps or sheets of sealing membrane material being appropriately shaped or sized for various portions of the face wherein the separate sections are separated into circumferential portions. Alternatively, numerous flaps can be arranged in echelon having various or similar stiffnesses so as to provide a more comfortable seal.

Additionally, each of the various masks and corresponding support systems having various contours, sizes, durometer, resilience, and shapes. Additionally, each such variation to the nasal or mouth interfaces can each be formed so as to accept a uniform input adapter 300.

The above embodiments can be formed of various materials include silicone materials, plastics, and the like. Furthermore, the durometer of each of the materials can be varied. For example, the durometer of the outer shell can be more pliable compared to the internal support structure. Alternatively, the thicknesses of the internal support structure can vary to provide the necessary mechanical coupling of a force being applied to the attachment means.

The above description is merely illustrative. Having thus described several aspects of at least one embodiment of this invention including the preferred embodiments, it is to be appreciated that various alterations, modifications, and improvements will readily occur to those skilled in the art. Such alterations, modifications, and improvements are intended to be part of this disclosure, and are intended to be within the scope of the invention which is defined by the appended claims. Accordingly, the foregoing description and drawing are by way of example only.

## Claims

1. A positive airway interface system comprising:
a positive air pressure adapter (300) that can connect to a positive air pressure source;
a mouth interface (100) connected to the positive air pressure adapter (300), the mouth interface comprising:
an internal support structure (130);
an outer shell (112) disposed over and suspended by the internal support structure (130) and being configured to adopt over and around the user's mouth; and
one or more malleable sealing membranes (150) disposed about a user interface aperture (118);
a nasal interface (200) being fluidly connected to the positive air pressure source, the nasal interface (200) comprising:
a pair of extensions (250) extending upwardly from the positive air pressure adapter (300); and
a pair of nasal pillows (270), wherein at least one nasal pillow is affixed to, and corresponds with, each of the extensions (250);
a plurality of attachment means on each of the nasal interface (200) and the mouth interface (100) wherein the attachment means comprises an attachment mechanism (170) extending through the outer shell (112) and connecting to the internal support structure (130), and wherein in use a force on the attachment means causes the nasal interface (200) to engage with the nares of a user, and the force causes the mouth interface (100) to sealingly engage around a mouth of the user.

2. The positive airway interface system of claim 1, wherein the sealing membrane (150) is affixed to the internal support structure (130) of the mouth interface by interferingly engaging a channel provided about a circumference of a facial aperture of the mouth interface (100).

3. The positive airway interface system of claim 1, comprising a removable heat moisture exchange (400) disposed within the positive air pressure adapter (300).

4. The positive airway interface system of claim 2, comprising one or more CO₂ washout vents (330) being provided through the positive air pressure adapter (300).

5. The positive airway interface system of claim 4, wherein the one or more CO₂ washout vents (330) are provided in a removable insert which covers a front aperture of the positive air pressure adapter (300).

6. The positive airway interface system of claim 2, comprising an anti-asphyxia valve (350) provided through the positive air pressure adapter (300).

7. The positive airway interface system of claim 1, wherein the attachment mechanism (170) is mechanically coupled to the internal support structure (130) of the mouth interface (100).

8. The positive airway interface system of claim 1, wherein the attachment mechanisms (170) are formed of a plurality of circular grommets (170) being configured to affix to corresponding loops of a headgear assembly.

9. The positive airway interface system of claim 7, comprising:
a first extension (250) disposed on a first end of the nasal interface; and
a second extension (250) disposed on a second end of the nasal interface.

10. The positive airway interface system of claim 9, comprising one or more strap assemblies (50), each strap assembly (50) comprising a first strap assembly (54) configured to couple to the mouth attachment mechanisms (100) and a second strap assembly (58) configured to couple to the first and second nasal attachment mechanisms (200), the one or more strap assemblies configured to position and provide a multi-dimensional sealing force about the positive airway interface system and the user's face.

11. The positive airway interface system of claim 1, comprising:
a first malleable sealing membrane (150) extending inwardly into an internal cavity of the facial mask from an external edge of the internal support structure (130); and
a second malleable sealing membrane (154) extending inwardly into the internal cavity of the facial mask from an internal edge of the internal support structure (130).

12. The positive airway interface system of claim 1, comprising a support membrane extending from a first point of an outer circumference of the facial mask to a second point of the outer circumference of the facial mask, the support membrane configured to rest on a maxilla portion of the user's face between the user's nose and mouth.

13. The positive airway interface system of claim 1, wherein the internal support structure (130) is formed of interconnected truss or web features.

14. The positive airway interface system of claim 13, wherein the plurality of attachment means of the mouth interface internal support structure (130) directly attach to the interconnected truss or web features of the internal support structure (130).

## Patentansprüche

1. Positives Atemwegsschnittstellensystem, Folgendes umfassend:
einen Positivluftdruckadapter (300), der mit einer Positivluftdruckquelle verbunden werden kann;
eine Mundschnittstelle (100), die mit dem Positivluftdruckadapter (300) verbunden ist, wobei die Mundschnittstelle Folgendes umfasst:
eine innere Stützstruktur (130);
eine äußere Hülle (112), die über der inneren Stützstruktur (130) angeordnet und von dieser aufgehängt ist und konfiguriert ist, um über und um den Mund des Benutzers herum zu passen; und
eine oder mehrere formbare Dichtungsmembranen (150), die um eine Öffnung (118) der Benutzerschnittstelle angeordnet sind;
eine nasale Schnittstelle (200), die fluidisch mit der Positivluftdruckquelle verbunden ist, wobei die nasale Schnittstelle (200) Folgendes umfasst:
ein Paar Verlängerungen (250), die sich von dem Positivluftdruckadapter (300) nach oben erstrecken; und
ein Paar nasaler Kissen (270), wobei zumindest ein nasales Kissen an jeder der Verlängerungen (250) befestigt ist und diesen entspricht;
eine Vielzahl von Befestigungsmitteln an jeder der nasalen Schnittstelle (200) und der Mundschnittstelle (100),
wobei die Befestigungsmittel einen Befestigungsmechanismus (170) umfassen, der sich durch die äußere Hülle (112) erstreckt und sich mit der inneren Stützstruktur (130) verbindet, und wobei im Gebrauch eine Kraft auf die Befestigungsmittel bewirkt, dass die nasale Schnittstelle (200) mit den Nasenlöchern eines Benutzers in Eingriff kommt, und die Kraft bewirkt, dass die Mundschnittstelle (100) dichtend um einen Mund des Benutzers in Eingriff kommt.

2. Positives Atemwegsschnittstellensystem nach Anspruch 1, wobei die Dichtungsmembran (150) an der inneren Stützstruktur (130) der Mundschnittstelle durch störendes Eingreifen in einen Kanal, der um einen Umfang einer Gesichtsöffnung der Mundschnittstelle (100) bereitgestellt wird, befestigt ist.

3. Positives Atemwegsschnittstellensystem nach Anspruch 1, umfassend einen abnehmbaren Wärme-Feuchtigkeitsaustausch (400), der innerhalb des Positivluftdruckadapters (300) angeordnet ist.

4. Positives Atemwegsschnittstellensystem nach Anspruch 2, umfassend eine oder mehrere CO₂-Auswaschentlüftungen (330), die durch den Positivluftdruckadapter (300) bereitgestellt sind.

5. Positives Atemwegsschnittstellensystem nach Anspruch 4, wobei die eine oder mehreren CO₂-Auswaschentlüftungen (330) in einem abnehmbaren Einsatz bereitgestellt sind, der eine vordere Öffnung des Positivluftdruckadapters (300) abdeckt.

6. Positives Atemwegsschnittstellensystem nach Anspruch 2, umfassend ein Antierstickungsventil (350), das durch den Positivluftdruckadapter (300) bereitgestellt ist.

7. Positives Atemwegsschnittstellensystem nach Anspruch 1, wobei der Befestigungsmechanismus (170) mechanisch mit der inneren Stützstruktur (130) der Mundschnittstelle (100) gekoppelt ist.

8. Positives Atemwegsschnittstellensystem nach Anspruch 1, wobei die Befestigungsmechanismen (170) aus einer Vielzahl von kreisförmigen Ösen (170) gebildet sind, die konfiguriert sind, um an entsprechenden Schlaufen einer Kopfbedeckungsanordnung befestigt zu werden.

9. Positives Atemwegsschnittstellensystem nach Anspruch 7, Folgendes umfassend:
eine erste Verlängerung (250), die an einem ersten Ende der nasalen Schnittstelle angeordnet ist; und
eine zweite Verlängerung (250), die an einem zweiten Ende der nasalen Schnittstelle angeordnet ist.

10. Positives Atemwegsschnittstellensystem nach Anspruch 9, umfassend eine oder mehrere Gurtanordnungen (50), wobei jede Gurtanordnung (50) eine erste Gurtanordnung (54) umfasst, die konfiguriert ist, um mit den Mundbefestigungsmechanismen (100) gekoppelt zu werden, und eine zweite Gurtanordnung (58), die konfiguriert ist, um mit den ersten und zweiten nasalen Befestigungsmechanismen (200) gekoppelt zu werden, wobei die eine oder mehreren Gurtanordnungen konfiguriert sind, um eine mehrdimensionale Dichtkraft um das positive Atemwegsschnittstellensystem und das Gesicht des Benutzers zu positionieren und bereitzustellen.

11. Positives Atemwegsschnittstellensystem nach Anspruch 1, Folgendes umfassend:
eine erste formbare Dichtungsmembran (150), die sich von einem äußeren Rand der inneren Stützstruktur (130) nach innen in einen inneren Hohlraum der Gesichtsmaske erstreckt; und
eine zweite formbare Dichtungsmembran (154), die sich von einem inneren Rand der inneren Stützstruktur (130) nach innen in den inneren Hohlraum der Gesichtsmaske erstreckt.

12. Positives Atemwegsschnittstellensystem nach Anspruch 1, umfassend eine Stützmembran, die sich von einem ersten Punkt eines Außenumfangs der Gesichtsmaske zu einem zweiten Punkt des Außenumfangs der Gesichtsmaske erstreckt, wobei die Stützmembran konfiguriert ist, um auf einem Oberkieferabschnitt des Gesichts des Benutzers zwischen Nase und Mund des Benutzers zu liegen.

13. Positives Atemwegsschnittstellensystem nach Anspruch 1, wobei die innere Stützstruktur (130) aus miteinander verbundenen Binder- oder Netzmerkmalen gebildet ist.

14. Positives Atemwegsschnittstellensystem nach Anspruch 13, wobei die Vielzahl von Befestigungsmitteln der inneren Stützstruktur (130) der Mundschnittstelle direkt an den miteinander verbundenen Binder- oder Netzmerkmalen der inneren Stützstruktur (130) befestigt ist.

## Revendications

1. Système d'interface positive avec les voies respiratoires comprenant :
un adaptateur de pression d'air positive (300) qui peut se raccorder à une source de pression d'air positive ;
une interface buccale (100) raccordée à l'adaptateur de pression d'air positive (300), l'interface buccale comprenant :
une structure de support interne (130) ;
une coque externe (112) disposée par-dessus et suspendue par la structure de support interne (130) et qui est conçue pour s'adapter par-dessus et autour de la bouche de l'utilisateur ;
et
une ou plusieurs membranes d'étanchéité malléables (150) disposées autour d'une ouverture d'interface avec l'utilisateur (118) ;
une interface nasale (200) raccordée de manière fluidique à la source de pression d'air positive, l'interface nasale (200) comprenant :
une paire d'extensions (250) s'étendant vers le haut depuis l'adaptateur de pression d'air positive (300) ; et
une paire de coussinets narinaires (270), au moins un coussinet narinaire étant fixé, et correspondant, à chacune des extensions (250) ;
une pluralité de moyens de fixation sur chacune de l'interface nasale (200) et de l'interface buccale (100)
les moyens de fixation comprenant un mécanisme de fixation (170) s'étendant à travers la coque externe (112) et se raccordant à la structure de support interne (130), et, lors de l'utilisation, une force sur les moyens de fixation provoquant l'application de l'interface nasale (200) aux narines de l'utilisateur, et la force provoquant l'application étanche de l'interface buccale (100) autour de la bouche de l'utilisateur.

2. Système d'interface positive avec les voies respiratoires selon la revendication 1, dans lequel la membrane d'étanchéité (150) est fixée à la structure de support interne (130) de l'interface buccale par application avec interférence d'un conduit formé autour d'une circonférence d'une ouverture faciale de l'interface buccale (100).

3. Système d'interface positive avec les voies respiratoires selon la revendication 1, comprenant un échange de chaleur et d'humidité amovible (400) placé à l'intérieur de l'adaptateur de pression d'air positive (300).

4. Système d'interface positive avec les voies respiratoires selon la revendication 2, comprenant un ou plusieurs évents d'expiration de CO₂ (330) formés à travers l'adaptateur de pression d'air positive (300).

5. Système d'interface positive avec les voies respiratoires selon la revendication 4, dans lequel le ou les évents d'expiration de CO₂ (330) sont formés dans un insert amovible qui recouvre une ouverture avant de l'adaptateur de pression d'air positive (300).

6. Système d'interface positive avec les voies respiratoires selon la revendication 2, comprenant une valve antiasphyxie (350) formée à travers l'adaptateur de pression d'air positive (300).

7. Système d'interface positive avec les voies respiratoires selon la revendication 1, dans lequel le mécanisme de fixation (170) est mécaniquement couplé à la structure de support interne (130) de l'interface buccale (100).

8. Système d'interface positive avec les voies respiratoires selon la revendication 1, dans lequel les mécanismes de fixation (170) sont formés d'une pluralité d'oeillets circulaires (170) qui sont conçus pour se fixer à des boucles correspondantes d'un ensemble couvre-chef.

9. Système d'interface positive avec les voies respiratoires selon la revendication 7, comprenant :
une première extension (250) disposée sur une première extrémité de l'interface nasale ; et
une seconde extension (250) disposée sur une seconde extrémité de l'interface nasale.

10. Système d'interface positive avec les voies respiratoires selon la revendication 9, comprenant un ou plusieurs ensembles sangles (50), chaque ensemble sangle (50) comprenant un premier ensemble sangle (54) conçu pour se coupler aux mécanismes de fixation buccaux (100) et un second ensemble sangle (58) conçu pour se coupler au premier et au second mécanisme de fixation nasaux (200), le ou les ensembles sangles étant conçus pour se positionner et exercer une force d'étanchéification multidimensionnelle autour du système d'interface positive avec les voies respiratoires et du visage de l'utilisateur.

11. Système d'interface positive avec les voies respiratoires selon la revendication 1, comprenant :
une première membrane d'étanchéité malléable (150) s'étendant vers l'intérieur dans une cavité interne du masque facial depuis un bord externe de la structure de support interne (130) ; et
une seconde membrane d'étanchéité malléable (154) s'étendant vers l'intérieur dans la cavité interne du masque facial depuis un bord interne de la structure de support interne (130).

12. Système d'interface positive avec les voies respiratoires selon la revendication 1, comprenant une membrane de support s'étendant d'un premier point d'une circonférence externe du masque facial à un second point de la circonférence externe du masque facial, la membrane de support étant conçue pour reposer sur une partie du maxillaire du visage de l'utilisateur entre le nez et la bouche de l'utilisateur.

13. Système d'interface positive avec les voies respiratoires selon la revendication 1, dans lequel la structure de support interne (130) est formée d'éléments de treillis ou de toile interconnectés.

14. Système d'interface positive avec les voies respiratoires selon la revendication 13, dans lequel la pluralité de moyens de fixation de la structure de support interne d'interface buccale (130) se fixe directement sur les éléments de treillis ou de toile interconnectés de la structure de support interne (130).
